# EUROPEAN PATENT APPLICATION

(11) **EP 4 455 077 A1**
(43) Date of publication of application: **30.10.2024**
(21) Application number: 22910809.7
(22) Date of filing: 01.12.2022
(51) Int. Cl.: C01B 21/064, A61K 8/19, A61Q 1/02, A61Q 1/12

(54) **HEXAGONAL BORON NITRIDE POWDER AND METHOD FOR PRODUCING SAME**

(30) Priority: 21.12.2021 JP 2021206699
(71) Applicant: TOKUYAMA CORPORATION, Yamaguchi 745-8648 (JP)
(72) Inventor: ABURATANI, Makoto, Shunan-shi, Yamaguchi 745-8648 (JP)
(74) Representative: Kraus & Lederer PartGmbB
(86) International application number: PCT/JP2022/044322
(87) International publication number: WO 2023/120092

(57) **Abstract**

There is provided a hexagonal boron nitride powder, having: a particle diameter D95 of 70 µm or less; a dynamic friction coefficient (MIU) of 0.50 or less; and a deviation of the dynamic friction coefficient (MMD) of 0.005 or less in a volume-based cumulative particle diameter distribution (a) as measured by a laser diffraction scattering method for the powder untreated with ultrasonic, the D95 being a 95% cumulative value in the volume-based cumulative particle diameter distribution (a) in a particle size distribution curve; and an average particle diameter (D50) of 3 to 15 um; a (D90-D10)/D50 of 1.30 or less; and a content of coarse particles of 2.0 volume% or less, the coarse particles being more than three times as large as the D50, in a volume-based cumulative particle diameter distribution (b) as measured by a laser diffraction scattering method for the powder treated with ultrasonic, the D10, the D50, and the D90 being a 10% cumulative value, a 50% cumulative value, and a 90% cumulative value, respectively, in the volume-based cumulative particle diameter distribution (b) in a particle size distribution curve.

## Description

### Technical Field:

The present invention relates to a hexagonal boron nitride powder (hereinafter, may be abbreviated as a "boron nitride powder" or a "h-BN powder".) More specifically, the present invention relates to a hexagonal boron nitride powder suitable for use in cosmetics and a method for producing the same.

### Background Art:

Inorganic materials such as talc, mica and kaolin, and resin materials such as nylon powder and polyethylene powder have been used in cosmetics. However, these materials are not always satisfactory in terms of usability and stability. For example, inorganic materials such as talc, mica and kaolin allegedly cause a change in odor, as their catalytic activity can contribute to the degradation of perfume and oil. Resin materials such as nylon powder and polyethylene powder are chemically stable but are poor in formability.

Hence, hexagonal boron nitride, which has a flattened shape and superior lubricity to other materials, is being used as a pigment for cosmetics. Cosmetics, which serve as a base for dispersing a color pigment, greatly affect usability, such as spreadability (i.e., the property of ensuring a smooth application on the skin surface) and lasting (i.e., the property of long lasting on the skin).

Patent Document 1 describes a boron nitride powder containing plate-like aggregates of stacked primary particles, each having a flattened shape with predetermined average major axis and thickness. This powder is obtained by reacting boric acid, urea and boron carbide so that oxygen and carbon are contained at a predetermined ratio.

Patent Document 2 describes a hexagonal boron nitride powder having predetermined average particle diameter, maximum particle diameter, and specific surface area, as well as predetermined graphitization index, mean friction coefficient, and deviation of the mean friction coefficient. This powder can ensure improved smoothness and reduced roughness. Patent Document 2 describes a method for producing the hexagonal boron nitride powder in accordance with specified first and second firing conditions.

Patent Document 3 proposes a boron nitride powder having predetermined water permeation rate and oil absorption, based on the findings that the oil absorption of a component powder of a cosmetic is closely related to the property of long "lasting" on the skin. The boron nitride powder of Patent Document 3 is produced by heating boric acid, urea and boron carbide in two stages.

Patent Document 4 proposes a boron nitride powder defined such that its elution amount of boron, average particle diameter, and content of particles with a specific particle diameter are not more than predetermined values. This powder has excellent "moist" and "slimy" textures while maintaining appropriate smoothness; thus, it is suitable for use in cosmetics. Patent Document 4 describes the mean friction coefficient and deviation of the mean friction coefficient of this boron nitride powder. Patent Document 4 also describes a method for producing a boron nitride powder, which includes two-stage firing, pulverization and washing.

Patent Document 5 proposes a hexagonal boron nitride powder having predetermined specific surface area, major axis of primary particles, and aspect ratio. This powder can be used to achieve a resin sheet with high thermal conductivity and high dielectric strength. Patent Document 5 describes a method for producing a hexagonal boron nitride powder by mixing boron oxide, a nitrogen-containing organic compound, and lithium carbonate at a predetermined weight ratio and heating the mixture.

Patent Document 6 describes a method for producing a hexagonal boron nitride powder with a low aspect ratio by disintegrating a raw material powder without substantially pulverizing primary particles. The raw material powder contains hexagonal boron nitride particles with an aspect ratio in a predetermined range and hexagonal boron nitride particles with an aspect ratio higher than a predetermined value.

### Prior Art Documents:

### Patent Documents:

Patent Document 1: JP-A-2012-176910
Patent Document 2: JP-A-2018-165241
Patent Document 3: WO2014/049956
Patent Document 4: WO2019/172440
Patent Document 5: WO2020/179662
Patent Document 6: WO2021/085223

### Summary of the Invention:

### Problems to be solved by the Invention:

An object of the present invention is to provide a cosmetic that contains a hexagonal boron nitride powder and ensures good smoothness, a moist texture (fits well with the skin), and reduced roughness. Another object of the present invention is to provide a method for producing the boron nitride powder.

### Means for solving the Problems:

The present inventors have found that: using a BN seed crystal prepared by the melamine method as a seed crystal is a key to produce a hexagonal boron nitride powder with a uniform particle diameter by the reduction nitridation method which involves heating a raw material mixture in a nitrogen atmosphere; and the thus-produced boron nitride powder with a uniform particle diameter has good smoothness, a moist texture (fits well with the skin), and reduced roughness. Based on these findings, the present invention has been completed.

The present invention encompasses the following inventions.
1. A hexagonal boron nitride powder, having:
   a particle diameter D95 of 70 µm or less; a dynamic friction coefficient (MIU) of 0.50 or less; and a deviation of the dynamic friction coefficient (MMD) of 0.005 or less in a volume-based cumulative particle diameter distribution (a) as measured by a laser diffraction scattering method for the powder untreated with ultrasonic, the D95 being a 95% cumulative value in the volume-based cumulative particle diameter distribution (a) in a particle size distribution curve; and
   an average particle diameter (D50) of 3 to 15 um; a (D90-D10)/D50 of 1.30 or less; and a content of coarse particles of 2.0 volume% or less, the coarse particles being more than three times as large as the D50, in a volume-based cumulative particle diameter distribution (b) as measured by a laser diffraction scattering method for the powder treated with ultrasonic, the D10, the D50, and the D90 being a 10% cumulative value, a 50% cumulative value, and a 90% cumulative value, respectively, in the volume-based cumulative particle diameter distribution (b) in a particle size distribution curve.
2. The hexagonal boron nitride powder according to 1 above, for use in a cosmetic.
3. A cosmetic including the hexagonal boron nitride powder according to 1 or 2 above.
4. A method for producing a hexagonal boron nitride powder, including reducing and nitriding a raw material mixture by heating in a nitrogen atmosphere, wherein
   the raw material mixture contains an oxygen-containing boron compound, an oxygen-containing alkaline earth metal compound, a carbon source compound, and a seed crystal (S), the seed crystal (S) being a BN seed crystal produced by a melamine method.
5. The method according to 4 above, wherein
   the raw material mixture has an atomic ratio (B/C ratio) of boron atoms (B) in the oxygen-containing boron compound to carbon atoms (C) in the carbon source compound of 0.70 to 2.00,
   the raw material mixture has a molar ratio (MO/B₂O₃) of the oxygen-containing alkaline earth metal compound (MO; M is an alkaline earth metal) to the oxygen-containing boron compound of 0.01 to 1.0 in terms of oxide, and
   the raw material mixture has an atomic ratio (B^{S}/C ratio) of boron atoms (B^{S}) in the BN seed crystal as the seed crystal (S) to the carbon atoms (C) in the carbon source compound of 0.01 to 13.
6. The method according to 4 or 5 above, wherein the raw material mixture is reduced and nitrided by heating at 1700°C to 2100°C.
7. The method according to any one of 4 to 6 above, including classifying a boron nitride powder obtained by reducing and nitriding the raw material mixture by a sieve.
8. The method according to any one of 4 to 7 above, wherein the melamine method includes heating a raw material mixture containing an oxygen-containing boron compound and a nitrogen-containing organic compound at 500°C to 1200°C in a nitrogen atmosphere.

### Effect of the Invention:

The hexagonal boron nitride powder of the present invention has a sharp particle size distribution and a uniform particle diameter, so that it is suitable for use in cosmetics. Further, the cosmetic containing the hexagonal boron nitride powder according to the present invention has good smoothness, a moist texture (fits well with the skin), and reduced roughness. The method for producing the boron nitride powder according to the present invention is capable of producing a hexagonal boron nitride powder with a sharp particle size distribution and a uniform particle diameter.

### Mode for Carrying out the Invention:

### <Hexagonal boron nitride powder>

### <D95>

A boron nitride powder of the present invention has a particle diameter D95 of 70 µm or less, preferably 65 µm or less, and more preferably 60 um or less in a volume-based cumulative particle diameter distribution (a) as measured by a laser diffraction scattering method for the powder untreated with ultrasonic. The D95 is a value for the boron nitride powder untreated with ultrasonic. If the D95 exceeds the aforementioned value, properties required of cosmetics, such as smoothness, a moist texture, and reduced roughness, deteriorate. In the present invention, the D95 of the boron nitride powder untreated with ultrasonic refers to a 95% cumulative value in the volume-based cumulative particle diameter distribution (a) in the particle size distribution curve.

### <MIU and MMD>

MIU (dynamic friction coefficient) is a parameter for quantifying the smoothness of cosmetics. A lower MIU value indicates better smoothness of cosmetics. Meanwhile, MMD (deviation of dynamic friction coefficient) is used to quantify the roughness of cosmetics. A lower MMD value indicates lower roughness of cosmetics.

The dynamic friction coefficient (MIU) of the boron nitride powder of the present invention is 0.50 or less, preferably 0.46 or less, and more preferably 0.43 or less. The boron nitride powder with a MIU of 0.50 or less can impart improved smoothness to a cosmetic.

The deviation of the dynamic friction coefficient (MMD) of the boron nitride powder of the present invention is 0.005 or less, preferably 0.004 or less, and more preferably 0.003 or less. The boron nitride powder with a MMD of 0.005 or less can achieve a cosmetic with suppressed roughness, that is, improved usability.

The "MIU (dynamic friction coefficient)" and the "MMD (deviation of dynamic friction coefficient)" as used herein are dimensionless numbers measured by a friction tester (manufactured by KATO TECH CO., LTD. under the trade name of KES-SE.) More specifically, the boron nitride powder is placed on artificial leather (manufactured by Idemitsu Technofine, Co., Ltd. under the trade name of SUPPLALE PBZ13001 BK) that simulates the skin, such that the powder is spread thinly in an amount of 0.5 mg/cm². Then, a (10-mm square silicon) sensor portion of the friction tester is applied onto the powder, thereby measuring the MIU and the MMD. The measurement conditions of the friction tester are set as follows: the sensitivity is H; the test table moving speed is 1 mm/second; and the static load is 25 gf. The measurement is performed once. The operation of preparing the specimen (by applying the boron nitride powder onto the artificial leather), followed by the measurement by the friction tester is repeated five times. The average of the five measurements is determined as the MIU/MMD of the boron nitride powder.

### <Primary particles>

The hexagonal boron nitride powder of the present invention contains primary particles and secondary particles in which the primary particles are aggregated. As such, for the measurement of the particle diameter of the primary particles, the powder needs to be treated with ultrasonic under certain conditions so that the secondary particles are almost completely broken and only the primary particles remain. In the present invention, the particles of the powder treated with ultrasonic under certain conditions may be referred to as the primary particles. The ultrasonic treatment is performed as follows: 1 g of the hexagonal boron nitride powder of the present invention is dispersed in 20 g of ethanol and is subjected to an ultrasonic homogenizer. The particle diameter distribution of the primary particles is a volume-based cumulative particle diameter distribution (b) as measured by a laser diffraction scattering method.

When a cosmetic containing the boron nitride powder of the present invention is applied to the skin, the boron nitride powder is disintegrated and slides due to the force of the cosmetic application (i.e., the shear force between the finger and the skin). On this account, in the present invention, the physical properties of the primary particles are a key consideration, and attention is also given to the particle diameter distribution of the primary particles.

The average particle diameter (D50) of the primary particles is 3 to 15 um, preferably 4 to 12 um. The average particle diameter (D50) as used herein refers to a volume-based 50% cumulative value.

If the D50 of the primary particles of the boron nitride powder is less than 3 um, the powder will have poor smoothness. If the D50 of the primary particles is more than 15 um, the powder will be too smooth to have any "moist" and "slimy" textures as a cosmetic and will be too glittery in appearance; thus, such a powder is not suitable as a cosmetic raw material.

The (D90-D10)/D50 of the primary particles is 1.30 or less, preferably 1.20 or less, and more preferably 1.10 or less. A smaller (D90-D10)/D50 indicates a sharper particle diameter distribution and a more uniform particle diameter, which lead to good smoothness and a "moist" texture. The lower limit is not particularly limited, but is usually not less than 0.60 for boron nitride powder.

The primary particles contain coarse particles that are more than three times as large as the D50 in an amount of 2.0 volume% or less, preferably 1.8 volume% or less, and more preferably 1.3 volume% or less. A smaller content of the coarse particles results in a "moist" texture and suppressed roughness.

In the present invention, the D10, D50 and D90 of the primary particles respectively refer to a 10% cumulative value (D10), a 50% cumulative value (D50), and a 90% cumulative value (D90) in the volume-based cumulative particle diameter distribution (b) in the particle size distribution curve.

The particle size distribution curve is calculated from the volume-based particle size distribution of measurement powder dispersed in ethanol after ultrasonic treatment, which is measured with a laser diffraction scattering particle diameter distribution analyzer. More specifically, 20 g of ethanol is placed in a 50-mL screw tube bottle as a dispersing agent, and 1 g of measurement powder is dispersed in the ethanol, followed by ultrasonic treatment. The ultrasonic treatment is performed at an amplitude of 40% for 20 minutes using an ultrasonic homogenizer (manufactured by Branson Ultrasonics Corporation under the trade name of SFX250) by placing the tip of a chip (20 kHz microchip 1/4 inch) at a distance of 1 to 5 mm from the bottom of the screw tube.

### <Cosmetic>

The above-described hexagonal boron nitride powder can be used in cosmetics. The present invention encompasses a cosmetic containing the hexagonal boron nitride powder. Examples of the cosmetic include foundation (such as powder foundation, liquid foundation, and cream foundation), face powder, point makeup, eye shadow, eyeliner, nail polish, lipstick, blusher, and mascara. Among them, the cosmetic of the present disclosure is particularly well adapted to powder foundation and face powder.

The content of the boron nitride powder in the cosmetic is, for example, preferably 0.1 to 70 mass%, more preferably 1 to 30 mass%, of the total amount of the cosmetic.

The cosmetic may contain other components, including: pigments such as colcothar, yellow iron oxide, black iron oxide, titanium oxide, silica, aluminum hydroxide, and mica; esters such as diisostearyl malate and glyceryl tri(2-ethylhexanoate); and oils such as petrolatum. Further examples include talc, silicone powder, urethane powder, methyl paraben, and sodium dehydroacetate.

### <Method for producing hexagonal boron nitride powder>

A method for producing the boron nitride powder according to the present invention includes reducing and nitriding a raw material mixture by heating in a nitrogen atmosphere in the presence of a seed crystal (S).

### <Seed crystal (S)>

The seed crystal (S) is a hexagonal boron nitride powder (hereafter, may be referred to as a BN seed crystal) produced by the melamine method. The melamine method is a method for producing a BN seed crystal by using a nitrogen-containing organic compound (described below) as a nitrogen source for the nitridation reaction of an oxygen-containing boron compound. BN seed crystal produced from any other nitrogen-containing organic compound than melamine may also be suitably used.

The seed crystal (S) suitable for use in the present invention has a specific surface area of 5 m²/g or more as measured by the single-point BET method of nitrogen adsorption. Considering that larger primary particles usually have a smaller specific surface area, primary particles with a specific surface area of less than 5 m²/g are not suitable for the seed crystal (S), as they may be equal to or larger than the primary particles of the intended BN seed crystal. In a case where the BN seed crystal has an extremely small primary particle diameter, its particle size distribution may not be measured accurately by a laser diffraction scattering method. Taking this into account, the size of the primary particle diameter of the seed crystal (S) is evaluated by the specific surface area.

The specific surface area of the seed crystal (S) is preferably 5 m²/g or more, more preferably 8 m²/g or more, and still more preferably 10 m²/g or more.

The upper limit is not particularly limited, but is preferably 200 m²/g or less, particularly preferably 100 m²/g or less, since a too large specific surface area results in poor handleability.

The seed crystal (S) can be produced by heating a raw material mixture containing an oxygen-containing boron compound and a nitrogen-containing organic compound in a nitrogen atmosphere.

Examples of the oxygen-containing boron compound include boric acid, boric anhydride (boron oxide), metaboric acid, perboric acid, sub-boric acid, sodium tetraborate, and sodium perborate.

Examples of the nitrogen-containing organic compound include melamine, ammeline, cyandiamide, and urea.

The raw material mixture may also contain an oxygen-containing alkaline earth metal compound. Examples of the oxygen-containing alkaline earth metal compound include magnesium carbonate (MgCO₃), calcium carbonate (CaCO₃), and calcium oxide (CaO). Two or more of these compounds may be used in combination.

The heating temperature is preferably 500°C to 1200°C, more preferably 600°C to 1100°C, and still more preferably 650°C to 1000°C. A lower heating temperature is more likely to result in a seed crystal (S) with a uniform primary particle diameter.

The heating time is preferably 5 to 20 hours, more preferably 6 to 15 hours. A longer heating time is more likely to result in a seed crystal (S) with a uniform primary particle diameter.

The raw material mixture used to produce the seed crystal (S) preferably has a boron/nitride (B/N) element ratio of 0.2 to 1, more preferably 0.25 to 0.5.

The seed crystal (S) thus produced may be used as it is, but is preferably disintegrated before use by a jet mill, a stone mill disintegrator or the like so as to facilitate uniform mixing with other raw materials to be used in the reduction nitridation step. In addition, the seed crystal (S) may also be subjected to acid washing, filtration, water washing, and drying before use for refinement.

The seed crystal (S) produced by the melamine method is a BN seed crystal with a uniform particle diameter. This is presumably because the reaction temperature (i.e., the temperature of formation of boron nitride) in the melamine method is as low as 500°C to 1200°C, resulting in the BN seed crystal with a uniform particle diameter. In contrast, the reduction nitridation method is presumably less likely to produce the BN seed crystal with a uniform particle diameter, because the reaction temperature is as high as 1400°C or more, at which the formation of boron nitride proceeds simultaneously with crystal growth.

In the reduction nitridation step, particle growth occurs with the seed crystal (S) as a nucleus. As such, using the seed crystal (S) with a uniform particle diameter makes it easier to achieve a boron nitride powder with a uniform primary particle diameter.

### <Reduction nitridation step>

The reduction nitridation step involves heating a raw material mixture in a nitrogen atmosphere.

The raw material mixture contains an oxygen-containing boron compound, an oxygen-containing alkaline earth metal compound, a carbon source compound, and the seed crystal (S), i.e., the boron nitride powder produced by the melamine method.

### <Oxygen-containing boron compound>

Examples of the oxygen-containing boron compound can include diboron trioxide (boron oxide), diboron dioxide, tetraboron trioxide, tetraboron pentoxide, borax, and anhydrous borax. Among them, diboron trioxide (B₂O₃) is preferred. It is industrially beneficial to use diboron trioxide as the boron compound, as it is less expensive. Two or more of the compounds may be used in combination as the boron compound.

### <Oxygen-containing alkaline earth metal compound>

Examples of the oxygen-containing alkaline earth metal compound can include oxides and carbonates of an alkaline earth metal such as calcium or magnesium. Specific examples can include magnesium oxide, calcium oxide, magnesium carbonate, calcium carbonate, magnesium hydrogencarbonate, calcium hydrogencarbonate, magnesium hydroxide, calcium hydroxide, magnesium nitrate, calcium nitrate, magnesium sulfate, calcium sulfate, magnesium phosphate, calcium phosphate, magnesium oxalate, and calcium oxalate. When a carbonate, such as magnesium carbonate or calcium carbonate, is used as the oxygen-containing alkaline earth metal compound, carbon dioxide is generated in the heating step, making a layer of the raw material mixture porous. This allows nitrogen gas to be easily infiltrated into the layer of the raw material mixture, helping the reduction nitridation reaction to proceed efficiently. Two or more of the compounds may be used in combination as the oxygen-containing alkaline earth metal compound.

### <Carbon source compound>

Examples of the carbon source compound include: amorphous carbon such as carbon black, activated carbon, and carbon fiber; crystalline carbon such as diamond, graphite, and nanocarbon; and pyrolytic carbon obtained by thermal decomposition of a monomer or a polymer. When carbon black is used as the carbon source compound, the resultant hexagonal boron nitride powder can have a particularly uniform particle diameter.

The carbon source compound is used to cause the reduction nitridation reaction in the heating step, so that the resultant boron nitride contributes to the growth of the seed crystal (S). When the carbon source compound is not used, the seed crystal (S) is dissolved in the oxygen-containing boron compound, contributing to the growth of another seed crystal (S). In such a case, when the seed crystal (S) has a large particle diameter, the resultant hexagonal boron nitride powder has a less uniform primary particle diameter, though its cause has not been identified. In order to obtain a boron nitride powder with a sufficiently uniform primary particle diameter, it is necessary to use the carbon source compound to cause the reduction nitridation reaction.

### <Composition ratio of raw material mixture in reduction nitridation step>

The raw material mixture in the reduction nitridation step preferably has an atomic ratio (B/C ratio) of boron atoms (B) in the oxygen-containing boron compound to carbon atoms (C) in the carbon source compound of 0.70 to 2.00, more preferably 0.71 to 1.70, and still more preferably 0.72 to 1.30. The carbon source compound is blended so that the B/C ratio falls within this range, resulting in a boron nitride powder with a uniform primary particle diameter. If the carbon source compound is used in a small amount, a uniform primary particle diameter is difficult to achieve. If the carbon source compound is used in a too large amount, unreacted carbon unfavorably remains in the boron nitride powder as a black foreign material.

The raw material mixture preferably contains the oxygen-containing alkaline earth metal compound (MO; M is an alkaline earth metal) and the oxygen-containing boron compound at a molar ratio (MO/B₂O₃) of 0.01 to 1.0, more preferably 0.03 to 0.8, still more preferably 0.05 to 0.6, and particularly preferably 0.08 to 0.4, in terms of oxide. The oxygen-containing alkaline earth metal compound, which serves as a growth aid for boron nitride particles, is blended so that the MO/B₂O₃ falls within this range, resulting in a boron nitride powder with an intended primary particle diameter that contributes to good smoothness and reduced roughness.

The atomic ratio (B^{S}/C ratio) of boron atoms (B^{S}) in the boron nitride powder (BN seed crystal) as the seed crystal (S) to the carbon atoms (C) in the carbon source compound is preferably 0.01 to 13, more preferably 0.1 to 10, and still more preferably 0.2 to 8. The seed crystal (S) is blended so that the B^{S}/C ratio falls within this range, thereby efficiently providing a boron nitride powder with a uniform primary particle diameter. If the seed crystal (S) is contained in a small amount, it does not have a positive effect as a seed crystal. If the seed crystal (S) is contained in a too large amount, it is difficult to achieve a uniform primary particle diameter, and productivity is disadvantageously reduced.

### <Heating step>

The reduction nitridation step involves heating the raw material mixture in a nitrogen atmosphere. The boron nitride (BN) powder may be produced from boron oxide (B₂O₃) or an oxygen-containing boron compound, which is boron oxide (B₂O₃) produced by thermal decomposition, and carbon (C) or pyrolytic carbon, which is carbon (C) produced by thermal decomposition, by the following reaction:

B₂O₃ + 3C + N₂ → 2BN + 3CO

This reaction is initiated at about 1400°C or more. The reaction temperature is preferably 1400°C to 1600°C, more preferably 1450°C to 1580°C. A lower reaction temperature leads to an increase in reaction time, while a higher reaction temperature makes it difficult to achieve a boron nitride powder with a uniform primary particle diameter. The holding time is preferably 1 to 10 hours, more preferably 2 to 8 hours. A shorter holding time makes it difficult to achieve a boron nitride powder with a uniform primary particle diameter.

After the reaction, the heating temperature is raised to 1700°C to 2100°C. With a rise in temperature, the crystallization of boron nitride particles proceeds, making the primary particle diameter larger.

The maximum temperature is more preferably 1730°C to 2000°C, still more preferably 1750°C to 1950°C. A lower maximum temperature does not allow the primary particles of the boron nitride powder to grow to a size suitable as a cosmetic, while a higher maximum temperature causes the primary particles to grow excessively to a size larger than that suitable as a cosmetic.

The heating after the reaction is preferably held for at least 0.5 (more preferably 1) consecutive hours at a temperature in a range of 1700°C to 2100°C (preferably 1730°C to 2000°C, more preferably 1750°C to 1950°C). A shorter holding time tends to lead to poor reproducibility of the primary particle diameter of the boron nitride powder, while a longer holding time leads to lower productivity. The holding time at the aforementioned temperature is preferably 7 hours or less, more preferably 4 hours or less.

Nitrogen gas, which is necessary as a nitrogen source for the reduction nitridation reaction, is preferably flow in a furnace to be supplied. The flow path for nitrogen gas may be designed so that the nitrogen gas is allowed to flow in contact with the raw material mixture placed in a furnace (e.g., to flow above the layer of the raw material mixture). The amount of flowing nitrogen gas is preferably 0.1 to 20 L/minute, more preferably 0.2 to 15 L/minute, and still more preferably 0.3 to 10 L/minute, per kg of the raw material mixture. With a smaller amount of nitrogen gas, the reduction nitridation reaction is not completed, so that unreacted carbon unfavorably remains in the boron nitride powder as a black foreign material. With an excessive amount of nitrogen gas, thermal efficiency is unfavorably reduced.

A container for the raw material mixture may be prepared, such as a carbon container, a boron nitridecoated carbon container, or a boron nitride sintered body container, and the container filled with the raw material mixture may be placed in a furnace. The container preferably has a shape that does not prevent a contact between the raw material mixture and flowing nitrogen gas.

The raw material mixture preferably fills the container to a height of 1 to 20 cm. If the layer of the filling raw material mixture has a low height, productivity is reduced. If the layer of the filling raw material mixture has a too high height, the raw material mixture at the bottom of the container remains unreacted, so that unreacted carbon unfavorably remains in the boron nitride powder as a black foreign material. The height of the layer of the filling raw material mixture is preferably 2 to 15 cm, more preferably 3 to 10 cm.

### <Washing step>

The reaction product obtained by the reduction nitridation step contains impurities and the like, other than hexagonal boron nitride, such as unreacted raw materials (B₂O₃, CaO). Such impurities and the like can be removed by washing the reaction product with acid, resulting in a high purity hexagonal boron nitride powder suitable for use in cosmetics.

The method of acid washing is not particularly limited, and any known method is applicable without limitation. For example, the reaction product obtained by the reduction nitridation step is manually placed in a container, to which dilute hydrochloric acid (5 to 20 mass% HCl) is added in an amount 3 to 10 times that of the reaction product, followed by stirring for 1 to 20 hours.

After the acid washing, the resultant product is washed with pure water for the removal of the acid. The pure water available for use in the washing is water with an electrical conductivity of 1 mS/m or less for the avoidance of secondary contamination by impurities. The washing is performed as follows: After filtration of the acid used for the acid washing, pure water is added in the same amount as the acid used, followed by another filtration. This operation of pure water washing and filtration is repeated until the filtrate is neutralized.

In particular, the hexagonal boron nitride powder for use in cosmetics is desired to conform to the Japanese Standards of Quasi-drug Ingredients 2021, which specify a purity of 95% or more, a pH of 5.0 to 8.0, an elution amount of boron of 20 ppm or less, and the like. As such, the washing needs to be performed to meet these standards.

After the washing, the resultant product may be subjected to solid-liquid separation, followed by drying. In such a case, the method of solid-liquid separation is not particularly limited, and can be carried out, for example, by decantation, a suction filter, a pressure filter, a rotation filter, a precipitator, or a combination thereof.

### <Drying step>

The hexagonal boron nitride powder obtained after the solid-liquid separation can be dried, for example, using a shelf-type dryer, a fluidized-bed dryer, a spray dryer, a rotary dryer, a belt dryer, a vacuum dryer, a vibration dryer, or a combination thereof. The atmosphere temperature in the dryer is preferably 50°C to 300°C, more preferably 100°C to 250°C. The drying time is not particularly specified, but is usually recommended to be 1 to 48 hours at the aforementioned temperature, as it is preferable for the hexagonal boron nitride powder to be dried to a moisture content as close to 0% as possible. Each of the washing, the solid-liquid separation, and the drying may be performed once or repeated a plurality of times by the same method or in combination with another method.

In the drying step, boron nitride may be hydrolyzed, possibly resulting in an elution amount of boron of more than 20 ppm. To suppress the hydrolysis of boron nitride, vacuum drying is preferred.

### <Classification step>

The hexagonal boron nitride powder is preferably classified for control of the D95. The method of classification is not particularly limited, and any known method is applicable without limitation, as long as the D95 can be controlled not to exceed a desired value. Specific examples include a vibrating sieving machine, a wet sieving machine, a wind classifier, a cyclone, and a liquid cyclone. In a case where wet classification is applied, this step is preferably conducted bofre the drying step.

### Examples

Hereinafter, the present invention will be described by way of Examples.

In the following examples, a low purity powder before washing is referred to as a nitride powder, and a high purity powder after the washing is referred to as a h-BN powder. A powder for preparing a seed crystal is denoted by (S).

### <Volume-based cumulative particle diameter distribution as measured by laser diffraction scattering method>

A sample untreated with ultrasonic and a sample treated with ultrasonic were prepared as described below. Each of the samples was measured for its h-BN powder particle size distribution by using a particle size distribution measuring device, MT3000 manufactured by NIKKISO CO., LTD. The solvent used was ethanol. The ethanol had a refractive index of 1.36, and hexagonal boron nitride had a refractive index of 1.74. The measurement was performed for 30 seconds.

### <Sample untreated with ultrasonic>

20 g of ethanol was placed in a 50-mL screw tube bottle as a dispersing agent, and 1 g of h-BN powder was dispersed in the ethanol, thereby preparing a measurement sample.

### <Sample treated with ultrasonic>

A measurement sample treated with ultrasonic was prepared as shown below. 20 g of ethanol was placed in a 50-mL screw tube bottle as a dispersing agent, and 1 g of h-BN powder was dispersed in the ethanol, thereby preparing the measurement sample. Then, the thus-prepared measurement sample was treated with ultrasonic at an amplitude of 40% for 20 minutes using an ultrasonic homogenizer, SONIFIER SFX250 manufactured by Branson Ultrasonics Corporation, by placing the tip of a chip (20 kHz microchip 1/4 inch) at a distance of 1 to 5 mm from the bottom of the screw tube. The resultant measurement sample treated with ultrasonic was measured for its particle size distribution.

### <Specific surface area>

The specific surface area was obtained by the BET method (the nitrogen adsorption single point method) using a specific surface area measuring device (Flow Sorb 2-2300 manufactured by SHIMADZU CORPORATION).

### <MIU and MMD>

The MIU (dynamic friction coefficient) and the MMD (deviation of dynamic friction coefficient) are dimensionless numbers measured by a friction tester (manufactured by KATO TECH CO., LTD. under the trade name of KES-SE). More specifically, the h-BN powder was placed on artificial leather (manufactured by Idemitsu Technofine, Co., Ltd. under the trade name of SUPPLALE PBZ13001 BK) that simulated the skin, such that the powder was spread thinly in an amount of 0.5 mg/cm². Then, a (10-mm square silicon) sensor portion of the friction tester was applied onto the powder, thereby measuring the MIU and the MMD. The measurement conditions of the friction tester were set as follows: the sensitivity was H; the test table moving speed was 1 mm/second; and the static load was 25 gf. The measurement was performed once. The operation of preparing the specimen (by applying the h-BN powder onto the artificial leather), followed by the measurement by the friction tester was repeated five times. The average of the five measurements was determined as the MIU/MMD of the h-BN powder.

### <Preparation of BN seed crystal (melamine 1)>

30 g of boron oxide (B₂O₃) as an oxygen-containing boron compound and 50 g of melamine as a nitrogen-containing organic compound were mixed together with a ball mill to prepare a raw material mixture (S). The thus-prepared raw material mixture (S) had a B/N of 0.36.

The raw material mixture (S) was placed in a batch furnace in which nitrogen gas was flowing so that the mixture (S) was heated in a heating step at 15°C/minute to a temperature of 1000°C and kept heated at 1000°C for 10 hours, thereby preparing a nitride powder (S). The thus-prepared nitride powder (S) was disintegrated by a stone grinding mill and washed with acid in a 5% aqueous hydrochloric acid solution, followed by filtration, water washing, and drying, thereby obtaining a h-BN powder (S) (melamine 1).

### <Preparation of BN seed crystal (melamine 2)>

30 g of boron oxide (B₂O₃) as the oxygen-containing boron compound and 50 g of melamine as the nitrogen-containing organic compound were mixed together with a ball mill to prepare a raw material mixture (S). The thus-prepared raw material mixture (S) had a B/N of 0.36.

The raw material mixture (S) was placed in a batch furnace in which nitrogen gas was flowing so that the mixture (S) was heated in the heating step at 15°C/minute to a temperature of 700°C and kept heated at 700°C for 10 hours, thereby preparing a nitride powder (S). The thus-prepared nitride powder (S) was disintegrated by a stone grinding mill and washed with acid in a 5% aqueous hydrochloric acid solution, followed by filtration, water washing, and drying, thereby obtaining a h-BN powder (S) (melamine 2).

### <Preparation of BN seed crystal (melamine 3)>

30 g of boron oxide (B₂O₃) as the oxygen-containing boron compound and 50 g of melamine as the nitrogen-containing organic compound were mixed together with a ball mill to prepare a raw material mixture (S). The thus-prepared raw material mixture (S) had a B/N of 0.36.

The raw material mixture (S) was placed in a batch furnace in which nitrogen gas was flowing so that the mixture (S) was heated in the heating step at 15°C/minute to a temperature of 1000°C and kept heated at 1000°C for 6 hours, thereby preparing a nitride powder (S). The thus-prepared nitride powder (S) was disintegrated by a stone grinding mill and washed with acid in a 5% aqueous hydrochloric acid solution, followed by filtration, water washing, and drying, thereby obtaining a h-BN powder (S) (melamine 3).

### <Preparation of BN seed crystal (melamine 4)>

50 g of boric acid (H₃BO₃) as the oxygen-containing boron compound and 50 g of urea as the nitrogen-containing organic compound were mixed together with a ball mill to prepare a raw material mixture (S). The thus-prepared raw material mixture (S) had a B/N of 0.49.

The raw material mixture (S) was placed in a batch furnace in which nitrogen gas was flowing so that the mixture (S) was heated in the heating step at 15°C/minute to a temperature of 800°C and kept heated at 800°C for 10 hours, thereby preparing a nitride powder (S). The thus-prepared nitride powder (S) was disintegrated by a stone grinding mill and washed with acid in a 5% aqueous hydrochloric acid solution, followed by filtration, water washing, and drying, thereby obtaining a h-BN powder (S) (melamine 4).

### <Preparation of BN seed crystal (reduction nitridation)>

50 g of boron oxide (B₂O₃) as the oxygen-containing boron compound, 8 g of calcium carbonate (CaCO₃) as an oxygen-containing alkaline earth metal compound, and 24 g of carbon black (C) as a carbon source compound were mixed together with a ball mill to prepare a raw material mixture (S). The thus-prepared raw material mixture had a B/C (molar ratio) of 0.72 and a CaO/B₂O₃ (molar ratio) of 0.11.

The raw material mixture (S) was placed in a batch furnace in which nitrogen gas was flowing so that the mixture (S) was heated in the heating step at 15°C/minute to a temperature of 1500°C and kept heated at 1500°C for 6 hours, thereby preparing a nitride powder (S). The thus-prepared nitride powder (S) was disintegrated by a stone grinding mill and washed with acid in a 5% aqueous hydrochloric acid solution, followed by filtration, water washing, and drying, thereby obtaining a h-BN powder (S) (reduction nitridation).

### <Example 1>

### <Raw material mixture>

50 g of boron oxide (B₂O₃) as an oxygen-containing boron compound, 8 g of calcium carbonate (CaCO₃) as an oxygen-containing alkaline earth metal compound, 25 g of carbon black (C) as a carbon source compound, and 40 g of the above-described seed crystal (melamine 1) were mixed together with a ball mill to prepare a raw material mixture. The thus-prepared raw material mixture had a B/C (atomic ratio) of 0.72, a CaO/B₂O₃ (molar ratio) of 0.11, and a B^{S}/C (atomic ratio) of 0.81.

### <Reduction nitridation step>

The raw material mixture thus prepared was placed in a batch furnace in which nitrogen gas was flowing so that the mixture (S) was heated at 1500°C for 6 hours and then at 1800°C for 2 hours, thereby producing a nitride powder.

### <Refinement step>

The thus-produced nitride powder was washed with acid in a 5% aqueous hydrochloric acid solution, followed by filtration, water washing, and vacuum drying at 200°C, thereby obtaining a h-BN powder.

### <Classification step>

The thus-obtained h-BN powder was classified using a vibrating sieve with an opening of 45 um. Table 1 shows the following properties of the resultant h-BN powder: the average particle diameter (D50), (D90-D10)/D50, and content of coarse particles that are more than three times as large as the D50 of the primary particles; the D95 of h-BN powder as a mixture of the primary particles and secondary particles in which the primary particles are aggregated; the dynamic friction coefficient (MIU) and the deviation of the dynamic friction coefficient (MMD).

### <Preparation of powder foundation>

The h-BN powder thus obtained was mixed with the following cosmetic components to prepare a cosmetic (powder foundation).

### (Component) (Blending amount %)

· h-BN powder 20.0
· Mica 15.0
· Synthetic phlogopite 12.0
· Ethylhexyl methoxycinnamate 8.0
· Vinyl dimethicone/methicone silsesquioxane crosspolymer 8.0
· Diphenyl dimethicone/vinyl diphenyl dimethicone/ silsesquioxane crosspolymer 8.0
· Nylon 12 3.0
· Silica 3.0
· Talc 3.0
· Acrylates crosspolymer 3.0
· Perfluorooctyl triethoxysilane 3.0
· Zinc oxide 3.0
· Polymethyl methacrylate polymer 3.0
· Silicone-treated colcothar (red iron oxide) 1.0
· Silicone-treated yellow iron oxide 0.6
· Silicone-treated black iron oxide 0.4
· Silicone-treated titanium oxide 6.0

### <Evaluation of powder foundation>

The resultant powder foundation was examined for its smoothness, roughness, and a moist texture; the results are shown in Table 1.

The aforementioned properties were evaluated by 20 research panelists specializing in cosmetic evaluation who used the products of the present invention and comparative products. The evaluation was made as follows: The product that was acceptable to less than 30% of the panelists was evaluated as ×; the product that was acceptable to not less than 30% and less than 60% of the panelists was evaluated as Δ; the product that was acceptable to not less than 60% and less than 80% of the panelists was evaluated as o; and the product that was acceptable to not less than 80% of the panelists was evaluated as ^{⊚}.

### <Example 2>

A h-BN powder was obtained in the same manner as in Example 1, except that the melamine 2 was used as a seed crystal. The particle size distribution, dynamic friction coefficient (MIU), and deviation of the dynamic friction coefficient (MMD) of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Example 3>

A h-BN powder was obtained in the same manner as in Example 1, except that the melamine 3 was used as a seed crystal. The particle size distribution, dynamic friction coefficient (MIU), and deviation of the dynamic friction coefficient (MMD) of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Example 4>

A h-BN powder was obtained in the same manner as in Example 1, except for the composition ratio of the raw material mixture and the firing temperature in the reduction nitridation step, which were changed as shown in Table 1. The particle size distribution, dynamic friction coefficient (MIU), and deviation of the dynamic friction coefficient (MMD) of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Example 5>

A h-BN powder was obtained in the same manner as in Example 1, except for the composition ratio of the raw material mixture and the firing temperature in the reduction nitridation step, which were changed as shown in Table 1. The particle size distribution, dynamic friction coefficient (MIU), and deviation of the dynamic friction coefficient (MMD) of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Example 6>

A h-BN powder was obtained in the same manner as in Example 1, except that the melamine 4 was used as a seed crystal. The particle size distribution, dynamic friction coefficient (MIU), and deviation of the dynamic friction coefficient (MMD) of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Example 7>

A h-BN powder was obtained in the same manner as in Example 1, except for the composition ratio of the raw material mixture, which was changed as shown in Table 1. The particle size distribution, dynamic friction coefficient (MIU), and deviation of the dynamic friction coefficient (MMD) of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Comparative Example 1>

A h-BN powder was produced under the same conditions as in Example 1, except for the absence of the seed crystal. The production conditions and the properties of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Comparative Example 2>

A h-BN powder was produced under the same conditions as in Comparative Example 1, except that the sieve had an opening of 90 um in the classification step. The production conditions and the properties of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Comparative Example 3>

A h-BN powder was obtained in the same manner as in Example 1, except for the use of the seed crystal (reduction nitridation) obtained by the reduction nitridation method. The particle size distribution, dynamic friction coefficient (MIU), and deviation of the dynamic friction coefficient (MMD) of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

### <Comparative Example 4>

A h-BN powder was obtained in the same manner as in Example 1, except for the composition ratio of the raw material mixture, which was changed as shown in Table 1. The particle size distribution, dynamic friction coefficient (MIU), and deviation of the dynamic friction coefficient (MMD) of the resultant h-BN powder are shown in Table 1.

Further, the h-BN powder was used to prepare powder foundation as in Example 1. The resultant powder foundation was evaluated for its properties in the same manner as in Example 1; the results are shown in Table 1.

**[Table 1-1]**

| | | | | | Ex. 1 | Ex. 2 | Ex. 3 | Ex. 4 | Ex. 5 | Ex. 6 | Ex. 7 |
|---|---|---|---|---|---|---|---|---|---|---|---|
| Raw material mixture | BN seed crystal | | Production method | - | Melamine 1 | Melamine 2 | Melamine 3 | Melamine 1 | Melamine 1 | Melamine 4 | Melamine 1 |
| | | | Specific surface area | m²/g | 12 | 86 | 15 | 12 | 12 | 23 | 12 |
| | Raw material composition ratio | | Seed crystal h-BN powder | g | 40 | 40 | 40 | 10 | 80 | 40 | 40 |
| | | | B₂O₃ | g | 50 | 50 | 50 | 60 | 20 | 50 | 50 |
| | | | C | g | 24 | 24 | 24 | 20 | 5 | 24 | 24 |
| | | | CaCO₃ | g | 8 | 8 | 8 | 25 | 12 | 8 | 4 |
| | | | MgCO₃ | g | - | - | - | - | - | - | 4 |
| | | | B/C (atomic ratio) | - | 0.72 | 0.72 | 0.72 | 1.04 | 1.38 | 0.72 | 0.72 |
| | | | CaO/B₂O₃ (molar ratio) | - | 0.11 | 0.11 | 0.11 | 0.29 | 0.42 | 0.11 | 0.12 |
| | | | B^{S}/C (atomic ratio) | - | 0.81 | 0.81 | 0.81 | 0.24 | 7.74 | 0.81 | 0.81 |
| Reduction nitridation | | | Firing temperature | °C | 1800 | 1800 | 1800 | 1900 | 1770 | 1800 | 1800 |
| Classification | | | Sieve opening | µm | 45 | 45 | 45 | 45 | 45 | 45 | 45 |
| h-BN powder | Particle size distribution | Treated with ultrasonic | Average particle diameter (D50) | µm | 7.7 | 7.7 | 7.8 | 10.5 | 7.1 | 7.6 | 7.2 |
| | | | (D90-D10)/D50 | - | 1.13 | 0.92 | 1.21 | 1.06 | 1.22 | 1.03 | 0.98 |
| | | | Rate of coarse particles more than 3 times as large as D50 | % | 1.2 | 0.9 | 1.5 | 1.3 | 1.1 | 1.2 | 1.0 |
| | | Untreated with ultrasonic | D95 particle diameter | µm | 54 | 57 | 58 | 57 | 53 | 62 | 55 |
| | Friction tester | | MIU | - | 0.42 | 0.38 | 0.44 | 0.39 | 0.43 | 0.37 | 0.41 |
| | | | MMD | - | 0.0035 | 0.0027 | 0.0040 | 0.0041 | 0.0036 | 0.0038 | 0.0036 |
| Foundation | | | Smoothness | - | ⊚ | ⊚ | ⊚ | ⊚ | ⊚ | ⊚ | ⊚ |
| | | | Roughness | - | ⊚ | ⊚ | ⊚ | ⊚ | ⊚ | ⊚ | ⊚ |
| | | | Moist texture | - | ⊚ | ⊚ | ⊚ | ⊚ | ⊚ | ⊚ | ⊚ |

| | | | | | | | | | | | |
|---|---|---|---|---|---|---|---|---|---|---|---|
| Ex.: Example | | | | | | | | | | | |

**[Table 1-2]**

| | | | | | Comp. Ex. 1 | Comp. Ex. 2 | Comp. Ex. 3 | Comp. Ex. 4 |
|---|---|---|---|---|---|---|---|---|
| Raw material mixture | BN seed crystal | | Production method | - | - | - | Reduction nitridation | Melamine 1 |
| | | | Specific surface area | m²/g | - | - | 5 | 12 |
| | Raw material composition ratio | | Seed crystal h-BN powder | g | - | - | - | 40 |
| | | | B₂O₃ | g | 50 | 50 | 50 | 20 |
| | | | C | g | 24 | 24 | 24 | - |
| | | | CaCO₃ | g | 8 | 8 | 8 | 8 |
| | | | MgCO₃ | g | - | - | - | - |
| | | | B/C (atomic ratio) | - | 0.72 | 0.72 | 0.72 | - |
| | | | CaO/B₂O₃ (molar ratio) | - | 0.11 | 0.11 | 0.11 | 0.28 |
| | | | B^{S}/C (atomic ratio) | - | 0 | 0 | 0.81 | - |
| Reduction nitridation | | | Firing temperature | °C | 1800 | 1800 | 1800 | 1800 |
| Classification | | | Sieve opening | µm | 45 | 90 | 45 | 45 |
| h-BN powder | Particle size distribution | Treated with ultrasonic | Average particle diameter (D50) | µm | 7.6 | 7.7 | 7.7 | 6.9 |
| | | | (D90-D10)/D50 | - | 1.33 | 1.35 | 1.35 | 1.32 |
| | | | Rate of coarse particles more than 3 times as large as D50 | % | 2.3 | 2.1 | 1.9 | 2.2 |
| | | Untreated with ultrasonic | D95 particle diameter | µm | 58 | 76 | 58 | 55 |
| | Friction tester | | MIU | - | 0.52 | 0.52 | 0.51 | 0.53 |
| | | | MMD | - | 0.0044 | 0.0056 | 0.0043 | 0.0065 |
| Foundation | | | Smoothness | - | ○ | ○ | ○ | ○ |
| | | | Roughness | - | Δ | Δ | ○ | Δ |
| | | | Moist texture | - | Δ | x | Δ | Δ |

| | | | | | | | | |
|---|---|---|---|---|---|---|---|---|
| Comp. Ex.: Comparative Example | | | | | | | | |

As shown in Table 1, the cosmetics using the hexagonal boron nitride powder of the present invention received high evaluation marks for smoothness, roughness, and a moist texture.

## Claims

1. A hexagonal boron nitride powder, having:
a particle diameter D95 of 70 µm or less; a dynamic friction coefficient (MIU) of 0.50 or less; and a deviation of the dynamic friction coefficient (MMD) of 0.005 or less in a volume-based cumulative particle diameter distribution (a) as measured by a laser diffraction scattering method for the powder untreated with ultrasonic, the D95 being a 95% cumulative value in the volume-based cumulative particle diameter distribution (a) in a particle size distribution curve; and
an average particle diameter (D50) of 3 to 15 um; a (D90-D10)/D50 of 1.30 or less; and a content of coarse particles of 2.0 volume% or less, the coarse particles being more than three times as large as the D50, in a volume-based cumulative particle diameter distribution (b) as measured by a laser diffraction scattering method for the powder treated with ultrasonic, the D10, the D50, and the D90 being a 10% cumulative value, a 50% cumulative value, and a 90% cumulative value, respectively, in the volume-based cumulative particle diameter distribution (b) in a particle size distribution curve.

2. The hexagonal boron nitride powder according to claim 1, for use in a cosmetic.

3. A cosmetic including the hexagonal boron nitride powder according to claim 1 or 2.

4. A method for producing a hexagonal boron nitride powder, including reducing and nitriding a raw material mixture by heating in a nitrogen atmosphere, wherein
the raw material mixture contains an oxygen-containing boron compound, an oxygen-containing alkaline earth metal compound, a carbon source compound, and a seed crystal (S), the seed crystal (S) being a BN seed crystal produced by a melamine method.

5. The method according to claim 4, wherein
the raw material mixture has an atomic ratio (B/C ratio) of boron atoms (B) in the oxygen-containing boron compound to carbon atoms (C) in the carbon source compound of 0.70 to 2.00,
the raw material mixture has a molar ratio (MO/B₂O₃) of the oxygen-containing alkaline earth metal compound (MO; M is an alkaline earth metal) to the oxygen-containing boron compound of 0.01 to 1.0 in terms of oxide, and
the raw material mixture has an atomic ratio (B^{S}/C ratio) of boron atoms (B^{S}) in the BN seed crystal as the seed crystal (S) to the carbon atoms (C) in the carbon source compound of 0.01 to 13.

6. The method according to claim 4 or 5, wherein the raw material mixture is reduced and nitrided by heating at 1700°C to 2100°C.

7. The method according to any one of claims 4 to 6, including classifying a boron nitride powder obtained by reducing and nitriding the raw material mixture by a sieve.

8. The method according to any one of claims 4 to 7, wherein the melamine method comprises heating a raw material mixture containing the oxygen-containing boron compound and a nitrogen-containing organic compound at 500°C to 1200°C in a nitrogen atmosphere.
